# EUROPEAN PATENT SPECIFICATION

(11) **EP 2 120 864 B1**
(45) Date of publication and mention of the grant of the patent: **22.03.2017**
(21) Application number: 07825668.2
(22) Date of filing: 12.11.2007
(51) Int. Cl.: A61K 9/00, A61K 35/74, A61K 47/36

(54) **A COMPOSITION FOR THE ADMINISTRATION OF BIOLOGICALLY ACTIVE PRINCIPLES IN GYNAECOLOGIC AND RECTAL FIELD AND USES THEREOF**
ZUSAMMENSETZUNG ZUR VERABREICHUNG VON BIOLOGISCH WIRKSAMEN WIRKSTOFFEN IN DER GYNÄKOLOGIE UND IM REKTALBEREICH UND IHRE VERWENDUNGEN
COMPOSITION SERVANT À L'ADMINISTRATION DE PRINCIPES BIOLOGIQUEMENT ACTIFS DANS LE CADRE GYNÉCOLOGIQUE OU RECTAL ET SES UTILISATIONS.

(30) Priority: 28.11.2006 IT MI20062286
(43) Date of publication of application: 25.11.2009
(73) Proprietor: Probiotical S.p.A., 28100 Novara (NO) (IT)
(72) Inventor: STROZZI, Gian Paolo, 28100 Novara (IT); MOGNA, Luca, 20124 Milano (IT)
(74) Representative: Hoffmann Eitle
(86) International application number: PCT/IB2007/003491
(87) International publication number: WO 2008/065492

(56) References cited:
- EP-A- 0 956 858
- EP-A- 1 262 192
- WO-A-84/04675
- WO-A-93/18758
- WO-A1-2005/060937
- DE-A1- 4 123 330
- JP-A- H05 262 657

## Description

The present invention relates to a composition for the administration of biologically active substances in a gynaecologic and rectal ambit, as well as the uses of said composition. Said composition presents particularly favourable technological and stability characteristics of the end product, above all as for the viscosity after the dissolution/resuspension of the same in a hydrophilic liquid medium which allows a better adhesion and a direct contact from bacteria to the treated mucosae. Said composition includes a specific viscosizing agent selected from the group of tara gum, which can play an important prebiotic function by contributing in a significant way to the correct growth and colonization of the probiotic culture, thus forming a perfect symbiotic system.

It is known that the compositions intended for the rectal or vaginal administration must have peculiar characteristics which are able to average a balanced and quantitatively correct delivery of the biologically active substance contained in said compositions. Most of the compositions, although having a varying water content, are however in a solid form, and are delivered as such.

In this case, the delivery of the biologically active substance can occur by melting, at the body temperature, of the whole components of the composition, or by dissolution or dispersion of said components in the vaginal secretion or in the liquid present in the rectum.

However, there are some cases in which the compositions above mentioned are introduced in the destination site in a liquid form, as a solution or dispersed system.

In this case, the composition may directly be in a liquid form, or it is necessary the addition of the composition itself to an adequate volume of liquid before the administration.

The presence of a biologically active substance in a composition with the characteristics above described, is often problematic, as the effectiveness of the same, after the rectal or vaginal introduction of a suspension or solution, tends to be limited over time. In fact, as most of the liquid administered tends to be discharged more or less quickly from the application seat, the effective contact time of said biologically active substance with the rectal or vaginal mucosa is sensibly reduced.

The use of a viscous formulation with an oily-hydrophobic base also presents some drawbacks. In fact, although it ensures a higher residence time of the active substance, for example a probiotic microorganism, it physically prevents the contact thereof since the oily-hydrophobic matrix forms an insulating film which avoids its direct contact with the treated mucosa.

In view of what above described, the residual quantity of biologically active substance remaining within the rectal or vaginal space is almost always insufficient to ensure a real effectiveness of the same, both when the action is carried out at a local level (like in most of the vaginal administrations) and when a good systemic absorption of the biologically active substance is desired (like in some typologies of rectal administrations).

The best current solution for the rectal or vaginal transport of biologically active substances is the administration of a composition in a solid form, which however has a more or less remarkable content of water or other liquid.

However, not all the biologically active substances can be introduced in solid compositions for rectal or vaginal purposes, to be introduced as such in the human body, as the quantity of water existing therein, often high, promotes the degradation thereof or however a more or less considerable chemical-physical alteration, in relatively short times.

Because of this, the time elapsing between the industrial preparation of the composition and the administration of the same is almost always the cause of a remarkable reduction of the effective titer of said active substance, up to levels often completely insufficient if compared to those typical of the therapeutic window.

A specific kind of biologically active substance, represented by the group including living microorganisms is noteworthy, with a particular reference to those with a probiotic and symbiotic valence.

In particular, the rectal administration of probiotic microorganisms presents, with respect to the oral one, the advantage that said microorganisms are not required to overcome the triple barrier (represented by the gastric juice, the bile secretion and the pancreatic one) and therefore are not subjected to unavoidable reductions, more or less remarkable, of their titer before reaching the intestine.

The best mode for storing said microorganisms until the time of administration, however, is in an anhydrous form, for example within a powdered formulation to be joined to a proper volume of liquid which is then capable of ensuring an effective and concrete transport of the microorganisms in the rectal or vaginal space.

The effectiveness of such composition is connected to the persistence, within the body space in question, of the probiotic microorganisms for sufficient times for expressing their clinical-healthy effect.

An effective persistence of said microorganisms would therefore lead to their actual integration within the intestinal and/or vaginal microflora, with a consequent ability of conducting in an advantageous way the metabolic and, more generally, the microbiologically manifested activity by said microflora.

In each case, it is not possible, at present, to assure a valid persistence of the probiotic microorganisms within the rectal and/or vaginal space after the administration of a composition with the characteristics above described, as the rapid discharging from the organism of the transport liquid of said microorganisms or their insulation in a oily-hydrophobic matrix would cause most of them to not have the time of colonizing the mucosa, with a consequent reduced effectiveness of said Formulation.

Therefore, there remains the need of being able to provide a composition in a solid form for an effective rectal and/or vaginal administration, after suspension or dissolution of the same in a proper hydrophilic liquid medium, of biologically active substances.

In particular, there remains the need of being able to provide a composition for the rectal and/or vaginal administration of biologically active substances, in particular microorganisms with a probiotic or symbiotic valence, which combine the characteristic of an actual and adequate stability of said substances within the composition itself with a concrete effectiveness of the composition during the administration.*
*EP 0 956 858 A1 discloses the use of lactobacilli for the preparation of a pharmaceutical composition for the treatment of vaginosis and vagonitics.

It is an object of the present invention to give an adequate answer to the need above pointed out.

This and other aims, which will result apparent from the following detailed description, have been attained by the Applicant which has unexpectedly found that, through the use of a proper quantity of at least a proper hydrophilic viscosizing agent selected from the group of tara gum, it is possible to prepare a composition for the rectal or vaginal administration of biologically active substances (preferably including microorganisms with a probiotic or symbiotic valence), capable of meeting the need above pointed out.

Said composition is in a solid form, advantageously in an anhydrous form. The composition is diluted before the administration, with a proper quantity of water or other physiologically compatible hydrophilic liquid medium.

In this way, a suspension or solution with such technological peculiarities to ensure the concrete transport of said substances within the body space of interest is obtained, as well as, above all, their persistence within the same and the direct contact with the treated mucosa.

Said technological peculiarities then allow an effective beneficial action of the composition, both when the biological action is manifested at a local level, and when it is necessary the systemic absorption of the substances themselves.

Therefore, a subject of the present invention is a composition for the rectal or vaginal administration of biologically active substances having the characteristics reported in the appended independent claim. Moreover, the composition for use in medicine and, in particular, in the gynaecologic or rectal ambit form another object of the present invention, as reported in the appended claims.

Preferred embodiments of the present invention are reported in the appended dependent claims.

Features and advantages of the present invention are pointed out in detail in the following description; moreover, they are further shown, by way of example, also within the enclosed Charts 1-4 and Tables 1-4, wherein:
- chart 1 shows the course ,over time, of the viscosity of a solution or suspension, kept at 25°C, obtained by addition of a composition according to the invention, including tara gum as a viscosizing agent and *Lactobacillus paracasei* as a biologically active substance, to 20 ml of water; the viscosity has been measured by using a rotational viscometer, or rotovis-cometer, with a SpR3 rotor and with an angular speed of 50 rpm, starting from the time of the resuspension/dissolution until the following 35 minutes, and is expressed in mPa·second (corresponding to 1 centipoise, cP); in this case the viscosizing agent is present at 0.9% (weight of the viscosizing agent/volume of resuspension or dissolution liquid, w/v);
- chart 2 shows the course over time of the viscosity of a solution or suspension, maintained at 38°C, through addition of the component used in the experiment of the chart 1 to 20 ml of water; the viscosity has been measured according to the same procedures of the chart 1; the viscosizing agent is present at 0.9% (w/v) ;
- chart 3 shows the course over time of the pH of 15 ml of a culture medium inoculated with 1% (v/v) of a *L. rhamnosus* in which the carbon and the energy sources are given by a guar gum. The pH measurements have been conducted from the time of the inoculum up to 24 hours from the inoculum. The positive control is given from a glucose containing medium. The negative control is given from a medium completely free of carbon and energy sources;
- chart 4 shows the course, over time, of the pH of 15 ml of a culture medium inoculated with 1% (v/v) of a *B. breve* in which the carbon and energy sources are given by a tara gum. The pH measurements have been conducted from the time of the inoculum up to 27 hours from the inoculum. The positive control is given from a glucose containing medium. The negative control is given from a medium completely free of carbon and energy sources;
- table 1 shows the viscosity values which have originated the chart 1;
- table 2 shows the viscosity values which have originated the chart 2;
- table 3 shows the pH values which have originated the chart 3;
- table 4 shows the pH values which have originated the chart 4.

The Applicant has found that, by introducing at least an opportune viscosizing agent selected from the group of tara gum in a composition, intended for the rectal or vaginal administration of at least a biologically active substance, it is possible to obtain, after resuspension or dissolution of said composition in an opportune volume of a physiologically compatible hydrophilic liquid medium, a viscosity of said liquid which is particularly suitable for the purpose above described and stable over time for a few hours.

In particular, said viscosity presents a peculiar course over time, by attesting on relatively low values in the times immediately after the dissolution/suspension of a composition according to the present invention and such to easily allow the suction of said liquid in an opportune cannula for the rectal and/or vaginal application, as well as pouring or mixing the same with other opportune components in a liquid or solid form.

With the passing of time, however, the tixotropic characteristics of the above suspension/solution cause the occurrence of a gradual increase of the viscosity. If the composition is adequately formulated the viscosity value does not reach excessive values, but such to allow a valid residence, in the intestinal or vaginal lumen, of the active substance existing therein for a sufficient number of hours, to ensure the colonization from the probiotic bacteria.

Preferably, the above viscosizing agent is added to a composition such that, after dissolution/resuspension in water or other proper liquid medium (generally called formulation), its percentage is ≥ 0.05% (w/v); advantageously, said percentage is between 0.2 and 20% (w/v), preferably from 0.4 to 5% (w/v); particularly preferred, said percentage is between 0.7 and 3% (w/v).

Said percentage is varying as a function of the specific viscosizing agent used and the physical-chemical peculiarities thereof, above all as for the tixotropic characteristics of the same when dissolved/resuspended in water or other proper liquid medium.

By mere way of absolutely not limiting example, there are reported three compositions in which the viscosizing agent is selected according to the criteria above stated, to be suspended/dissolved in 100 ml of water before the administration.

**Composition 1 (Reference Example)**

| | |
|---|---|
| Biologically active substance/s | 200 mg |
| Guar gum | 1.2 g |
| Excipients | 1.6 g |

**Composition 2**

| | |
|---|---|
| Biologically active substance/s | 250 mg |
| Tara gum | 1.1 g |
| Excipients | 1.85 g |

**Composition 3 (Reference Example)**

| | |
|---|---|
| Biologically active substance/s | 150 mg |
| Calcium alginate | 0.8 g |
| Excipients | 1.25 g |

Preferably, said compositions are formulated such that, after the dissolution/resuspension in an adequate volume of a liquid medium, they result isotonic if compared with the vaginal liquid or the rectal one. The pH of the solution/suspension obtained is generally between 0.5 and 8.5, preferably from 6.0 to 8.0, if said solution/suspension is intended for the introduction in the rectum, while it is generally between 3.5 and 7.0, preferably from 4.0 to 5.5 if said solution/suspension is intended for the introduction in the vaginal space.

By way of example, resuspension/dissolution volumes of a composition according to the invention, intended for the introduction within the rectum are generally ≥ 5 ml; preferably, between 8 and 70 ml, advantageously from 10 to 55 ml; particularly preferred, between 12 and 40 ml. Resuspension/dissolution volumes of a composition according to the invention, intended for the introduction within the vaginal space are generally ≥ 1 ml; preferably, between 1.5 and 10 ml, advantageously from 2 to 8 ml; particularly preferred, between 3 and 7 ml. Advantageously, said composition for vaginal purposes can also have a detergent purpose; in this case, resuspension volumes are generally ≥ 10 ml, preferably between 20 and 150 ml; particularly preferred, between 40 and 120 ml.

In a preferred embodiment of the invention, the biologically active substance of a composition according to the invention is selected from the group including living microorganisms physiologically compatible with the human body.

Preferably, said microorganisms are selected from the microorganisms group having a probiotic or symbiotic valence.

More preferably, said microorganisms with a probiotic valence are selected from the microbial group including the genera: *Bifidobacterium, Lactobacillus, Leuconostoc, Lactococcus, Streptococcus, Pediococcus, Propionibacterium, Bacillus, Enterococcus, Saccharomyces.*

For example, of the genus *Lactobacillus* the species: *L. pentosus, L. plantarum, L. casei* ssp. *casei, L. casei ssp. paracasei, L. rhamnosus, L. acidophilus, L. delbrueckii ssp. bulgaricus, L. delbrueckii ssp. lactis, L. fermentum, L. gasseri* have found use.

For example, of the genus *Bifidobacterium* the species: *B. longum, B. breve, B. bifidum, B. animalis, B. animalis* ssp. *lactis, B. adolescentis, B. pseudocatenulatum*, *B. catenulatum, B. infantis* have found use.

For example, of the genus *Lactococcus* the species: *L*. *lactis* and *L*. *lactis ssp. Lactis* have found use. For example, of the genus *Streptococcus* the species *S. thermophilus* has found use.

In a preferred embodiment of the invention, the composition includes from one to six strains, preferably four strains; advantageously, at least two bacterial probiotic strains selected from those above mentioned. In the table 5, by way of example, a group of microorganisms which find a valid application in the context of the present invention is reported.

All strains have been deposited according to the Budapest Treaty and are accessible to the public on request to the competent deposit Authority.

The composition according to the present invention finds a valid application for the preparation of a pharmaceutical formulation for use in the preventive and/or curative treatment of the rectum and the vagina; in particular, for use in the treatment of the infective pathologies of the rectum and the vagina by rectally or vaginally internal administration.

In another preferred embodiment of the invention, said at least one probiotic microorganism is added to a composition according to the invention, which also includes at least a prebiotic fiber, thus obtaining a symbiotic composition.

Said at least one prebiotic fiber is a molecule of a saccharide, generally oligo- or polysaccharide nature, usually soluble or at least partly soluble in water or in an aqueous solution where it can be used as a carbon and/or energy source from one or more probiotic microbial species having the required enzymatic complement for the hydrolysis of said fiber and for the consequent release of the constituting monosaccharide units.

Preferably, said prebiotic fiber is selected from the group including: fructo-oligosaccharides (FOS), galacto-oligosaccharides (GOS), trans-galacto-oligosaccharides (TOS), xylo-oligosaccharides (XOS), chitosan-oligosaccharides (COS), α-galactoside (such as raffinose, stachyose and so on), pectins, gums, partly hydrolized gums, inulin, psyllium, arabinogalactans, acacia, locust bean, oat, bamboo fibers, citrus fibers and, generally, fibers containing a soluble and an insoluble portions, in a varying ratio therebetween.

In a particularly preferred embodiment of the invention, the composition includes a mixture of two or more prebiotic fibers selected from those above mentioned.

The Applicant has found particularly advantageous the introduction of at least one prebiotic fiber to a composition having the features according to the present invention, intended for the rectal or vaginal administration of at least a probiotic microorganism, since above all the rectal environment is particularly poor from the point of view of the carbon and/or energy sources for the bacterial metabolism.

Advantageously, said prebiotic fiber can per se contribute, at least partly, to impart suitable viscosity characteristics, over time, to the suspension/solution intended for the introduction within the rectal or vaginal space, according to what has been above described.

In a preferred embodiment, the viscosizing agent in form of tara gum can play, in addition to its technological function above described, also an important prebiotic function by contributing in a significant way to the correct growth and colonization of the probiotic culture, thus forming a perfect symbiotic system.

In fact, in the experimental example reported in the Chart 3, table 3, it can be seen that a microorganism of the genus *Lactobacillus rhamnosus* is capable of using a gum, for example guar gum, as a prebiotic. Whereas, in the experimental example reported in the Chart 4, table 4, it can be seen that a microorganism of the genus *Bafidobacterium breve* is capable of using a gum, for example the tara gum, as a prebiotic.

As a not limiting example, there are reported two compositions according to the particularly preferred embodiments of the present invention intended for the rectal introduction, considering 20 ml as a dissolution/resuspension volume of the composition itself.

**Composition 1 (Reference Example)**

| | |
|---|---|
| *Lactobacillus rhamnosus* | 25 10⁹ CFU |
| Inulin | 0.80 g |
| Guar gum | 0.15 g |
| Calcium alginate | 0.10 g |
| Sodium chloride | 0.10 g |
| Magnesium citrate | 0.08 g |

**Composizione 2**

| | |
|---|---|
| *Bifidobacterium animals* ssp. *lactis* | 20.10⁹ CFU |
| Fructo-oligosaccharides (FOS) | 0.40 g |
| Inulin | 0.40 g |
| Tara gum | 0.18 g |
| Sodium chloride | 0.15 g |
| Calcium carbonate | 0.02 g |
| Hydroxypropylmethyl cellulose (HPMC) | 0,11 g |

By additional way of example, there are reported three compositions according to the particularly preferred embodiments of the present invention intended for the vaginal introduction, considering 4 ml as a dissolution/resuspension volume of the composition 3 and 80 ml as a dissolution/resuspension volume of the compositions 4 and 5.

**Composition 3**

| | |
|---|---|
| *Lactobacillus fermentum* | 20·10⁹ CFU |
| Arabinogalactan | 0.40 g |
| Galacto-oligosaccharides (GOS) | 0.40 g |
| Tara gum | 0.05 g |
| Sodium chloride | 0.025 g |
| Citric acid | 0.005 g |
| Microcrystalline cellulose | 0.03 g |

**Composition 4 (Reference Example)**

| | |
|---|---|
| *Lactobacillus paracasei* | 25·10⁹ CFU |
| Glucomannan | 0.90 g |
| Sodium chloride | 0.45 g |
| Citric acid | 0.04 g |
| Hydroxypropyl cellulose (HPC) | 0.48 g |

**Composition 5**

| | |
|---|---|
| *Bifidobacterium breve* | 20·10⁹ CFU |
| Fructo-oligosaccharides (FOS) | 0.40 g |
| Inulin | 0.40 g |
| Tara gum | 1.0 g |
| Sodium chloride | 0.45 g |
| Hydroxypropylmethyl cellulose (HPMC) | 0.40 g |

Preferably, the isotonic feature of said compositions, after dissolution/resuspension in the volume of used liquid, is assured with at least an excipient, generally a salt, selected from the group including the ions: chloride, iodide, carbonate in its mono- and di-basic forms, phosphate in its mono-, di- and tribasic forms, sulfate in its mono- and dibasic forms, nitrate, citrate, oxalate, gluconate, tartrate, lactate, acetate or mixtures thereof.

The cationic part of the salts above mentioned is generally selected from the group of ions including: sodium, calcium, magnesium, potassium, ammonium, manganese, copper, zinc, cobalt, iron ions or an opportune mixture of the same.

By additional way of example, in the compositions according to the present invention, one or more components selected from the group including: starches, modified starches, celluloses, hemicelluloses, modified celluloses, such as microcrystalline cellulose, hydroxypropylmethyl cellulose (HPMC), hydroxypropyl cellulose (HPC), hydroxypropylethyl cellulose (HPEC), methylcellulose, ethylcellulose, propylcellulose and other proper polymers, or opportune mixtures thereof, can be further additioned.

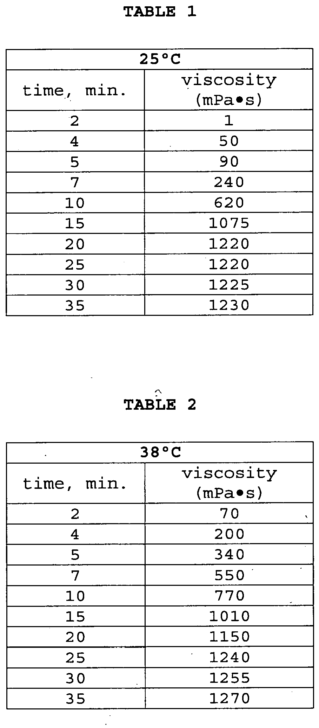

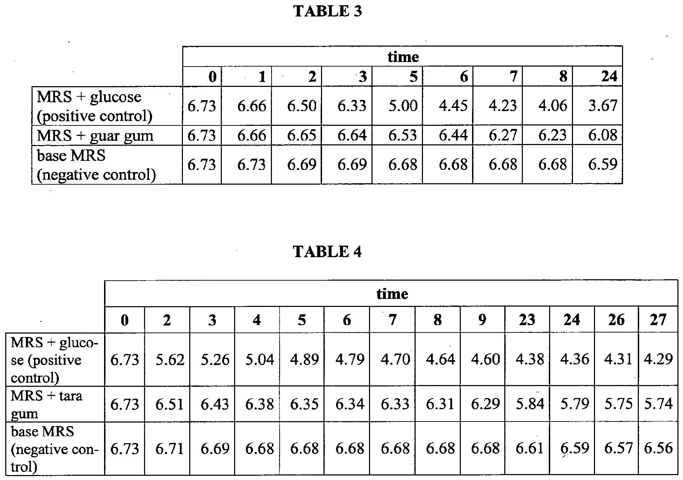

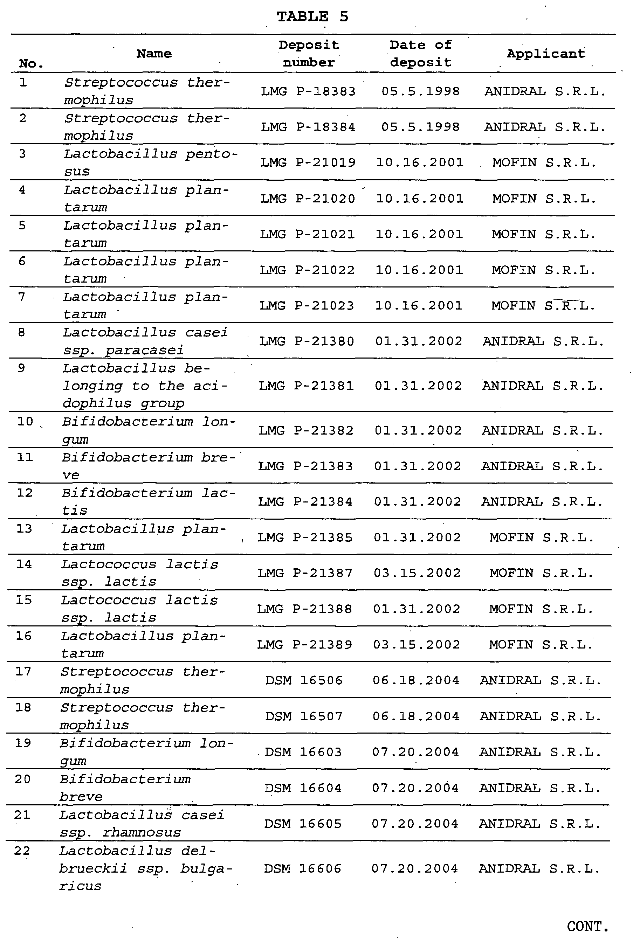

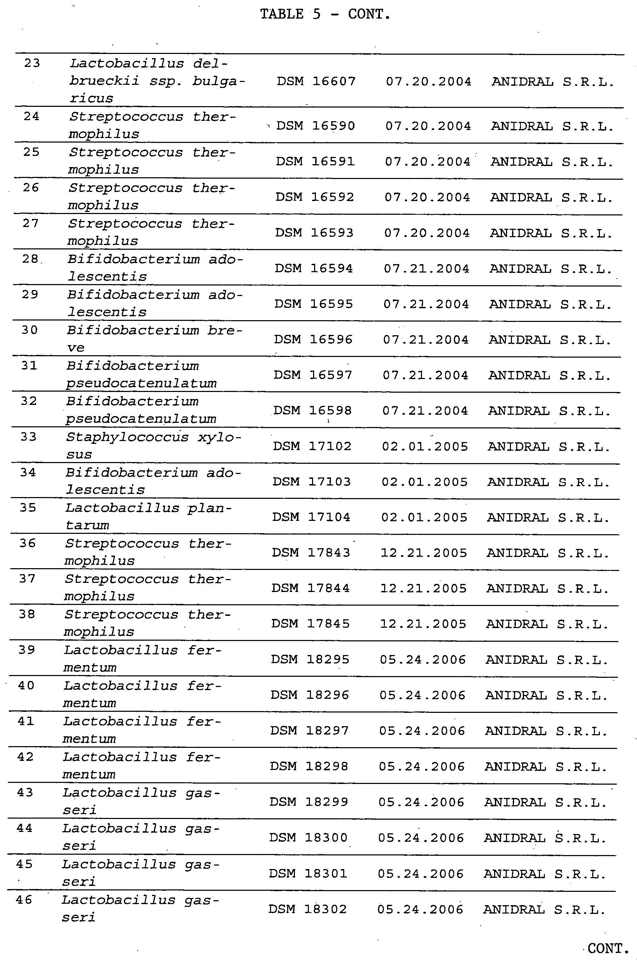

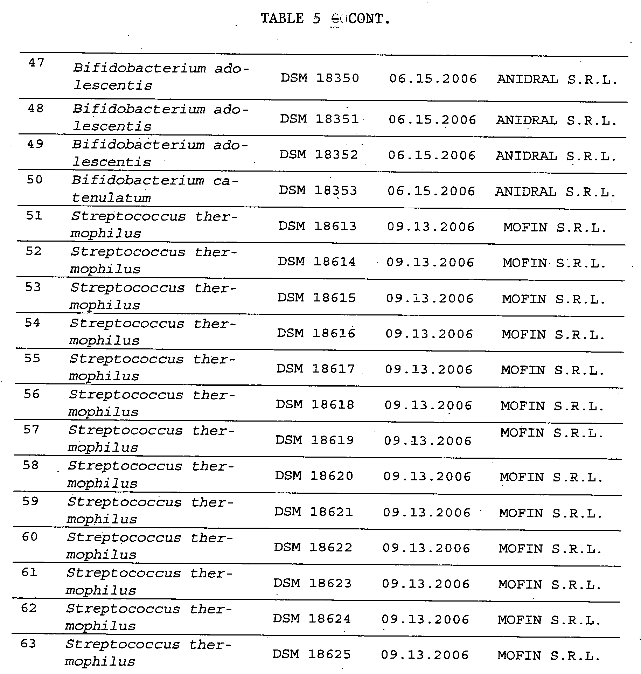

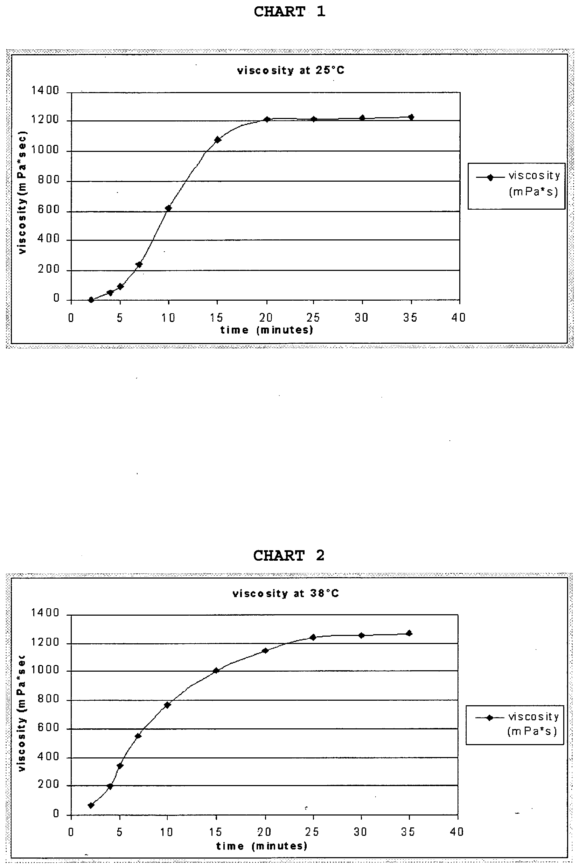

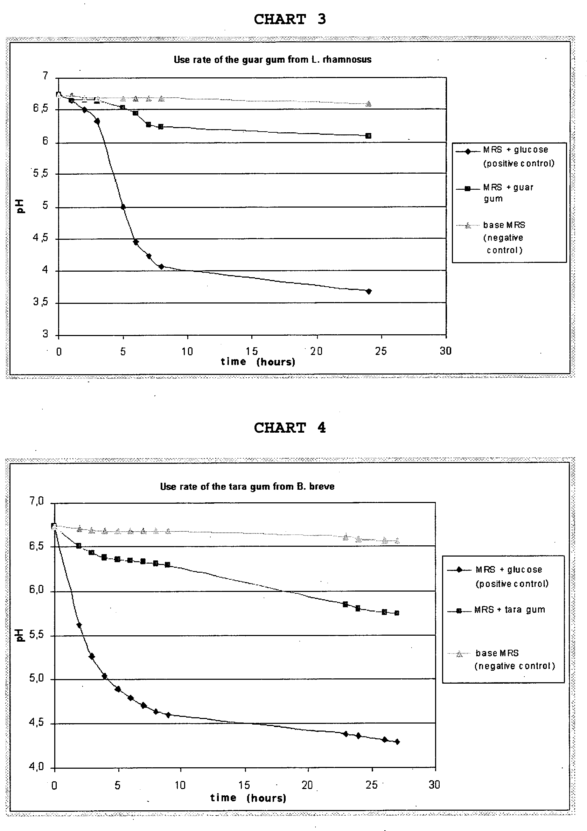

## Claims

1. Solid form composition including:
- at least a biologically active substance selected from the group including microorganisms; and
- at least a viscosizing agent selected from the group of tara gum for use in the prevention and/or curative treatment of the infective pathologies of the rectum and the vagina by rectally or vaginally internal administration.

2. The solid form composition for use according claim 1, wherein the microorganism is selected from the group including microorganisms having a probiotic or symbiotic valence.

3. The solid form composition for use according to claim 2, wherein said microorganism is selected from the group including the species: *Lactobacillus pentosus, Lactobacillus plantarum, Lactobacillus casei ssp. casei, Lactobacillus casei ssp. paracasei, Lactobacillus rhamnosus, Lactobacillus acidophilus, Lactobacillus delbrueckii ssp. bulgaricus, Lactobacillus delbrueckii ssp. lactis, Lactobacillus fermentum, Lactobacillus gasseri; Bifidobacterium longum, Bifidobacterium breve, Bifidobacterium bifidum, Bifidobacterium animalis, Bifidobacterium animalis ssp. lactis, Bifidobacterium adolescentis, Bifidobacterium pseudocatenulatum, Bifidobacterium catenulatum, Bifidobacterium infantis, Lactococcus lactis* and *Lactococcus lactis ssp. Lactis ; S. thermophilus.*

4. The solid form composition for use according to claim 3, wherein the composition includes from one to six strains, preferably four strains.

5. The solid form composition for use according to any one of the preceding claims, wherein said composition further includes at least a prebiotic fiber selected from the group consisting of fructo-oligosaccharides (FOS); galacto-oligosaccharides (GOS); trans-galacto-oligosaccharides (TOS) ; xylo-oligosaccharides (XOS) ; chitosan-oligosaccharides (COS) ; α-galactoside selected from raffinose, stachyose; pectins; gums; partly hydrolized gums; inulin; psyllium; arabinogalactans ; acacia, locust bean, oat, bamboo and citrus fibers.

6. Formulation including a solid form composition for use according to one or more of the claims 1 to 5 and a hydrophilic liquid medium.

7. Formulation including the solid form composition for use according to claim 6, wherein the viscosizing agent is present in a higher or equal percentage than 0.05% (w/v), based on the final volume of the formulation; preferably between 0.2% and 20% (w/v).

## Patentansprüche

1. Zusammensetzung in fester Form, enthaltend:
- wenigstens eine biologisch aktive Substanz ausgewählt aus der Gruppe enthaltend Mikroorganismen; und
- wenigstens ein viskositätseinstellendes Mittel ausgewählt aus der Gruppe bestehend aus Tara-Gummi zur Verwendung bei der präventiven und/oder curativen Behandlung von infektiven Pathologien des Rektum und der Vagina durch rektale oder intra-vaginale Verabreichung.

2. Zusammensetzung in fester Form zur Verwendung gemäß Anspruch 1, in der der Mikroorganismus aus der Gruppe aus Mikroorganismen mit einer probiotischen oder symbiotischen Valenz ausgewählt ist.

3. Zusammensetzung in fester Form zur Verwendung gemäß Anspruch 2, in der der Mikroorganismus aus der Gruppe der Species: Lactobacillus pentosus, Lactobacillus plantarum, Lactobacillus casei ssp. casei, Lactobacillus casei ssp. paracasei, Lactobacillus rhamnosus, Lactobacillus acidophilus, Lactobacillus delbrueckii ssp. bulgaricus, Lactobacillus delbrueckii ssp. lactis, Lactobacillus fermentum, Lactobacillus gasseri; Bifidobacterium longum, Bifidobacterium breve, Bifidobacterium bifidum, Bifidobacterium animalis, Bifidobacterium animalis ssp. lactis, Bifidobacterium adolescentis, Bifidobacterium pseudocatenulatum, Bifidobacterium catenulatum, Bifidobacterium infantis, Lactococcus lactis und Lactococcus lactis ssp. Lactis und S. thermophilus ausgewählt ist.

4. Zusammensetzung in fester Form zur Verwendung gemäß Anspruch 3, wobei die Zusammensetzung einen bis sechs Stämme, vorzugsweise vier Stämme enthält.

5. Zusammensetzung in fester Form zur Verwendung gemäß einem der vorhergehenden Ansprüche, wobei die Zusammensetzung außerdem wenigstens ein präbiotisches Fasermaterial enthält, ausgewählt aus der Gruppe aus: Fructo-oligosacchariden (FOS); Galacto-oligosacchariden (GOS); trans-Galactooligosacchariden (TOS); Xylo-oligosacchariden (XOS); Chitosan-oligosacchariden (COS); α-Galactosiden ausgewählt aus Raffinose, Stachyose; Pectinen; Gummen; teilweise hydrolysierten Gummen; Inulin; Psyllium; Arabinogalactanen; Acacia, Johannisbrot, Hafer, Bambus und Citrusfasern.

6. Formulierung enthaltend eine Zusammensetzung in fester Form zur Verwendung gemäß einem oder mehreren der Ansprüche 1 bis 5 und ein hydrophiles flüssiges Medium.

7. Formulierung enthaltend eine Zusammensetzung in fester Form zur Verwendung gemäß Anspruch 6, in der das viskositätseinstellende Mittel in einem Prozentsatz von größer oder gleich 0,05% (G/V), bezogen auf das Endgewicht der Formulierung enthalten ist; vorzugsweise zwischen 0,2% und 20% (G/V).

## Revendications

1. Composition sous forme solide contenant :
- au moins une substance biologiquement active choisie dans le groupe comprenant les micro-organismes ; et
- au moins un agent viscosifiant choisi dans le groupe de la gomme tara, pour son utilisation dans la prévention et/ou le traitement curatif des pathologies infectieuses du rectum et du vagin par administration interne par voie rectale ou vaginale.

2. Composition sous forme solide pour son utilisation selon la revendication 1, dans laquelle le micro-organisme est choisi dans le groupe comprenant les micro-organismes ayant une valence probiotique ou symbiotique.

3. Composition sous forme solide pour son utilisation selon la revendication 2, dans laquelle ledit micro-organisme est choisi dans le groupe comprenant les espèces : *Lactobacillus pentosus, Lactobacillus plantarum, Lactobacillus casei ssp. casei, Lactobacillus casei ssp. paracasei, Lactobacillus rhamnosus, Lactobacillus acidophilus, Lactobacillus delbrueckii ssp. bulgaricus, Lactobacillus delbrueckii ssp. lactis, Lactobacillus fermentum, Lactobacillus gasseri ; Bifidobacterium longum, Bifidobacterium breve, Bifidobacterium bifidum, Bifidobacterium animalis, Bifidobacterium animalis ssp. lactis, Bifidobacterium adolescentis, Bifidobacterium pseudocatenulatum, Bifidobacterium catenulatum, Bifidobacterium infantis, Lactococcus lactis* et *Lactococcus lactis ssp. lactis ; S. thermophilus.*

4. Composition sous forme solide pour son utilisation selon la revendication 3, dans laquelle la composition comprend de une à six souches, de préférence quatre souches.

5. Composition sous forme solide pour son utilisation selon l'une quelconque des revendications précédentes, dans laquelle ladite composition comprend en outre au moins une fibre prébiotique choisie dans le groupe constitué par les fructo-oligosaccharides (FOS) ; les galacto-oligosaccharides (GOS) ; les trans-galacto-oligosaccharides (TOS) ; les xylo-oligosaccharides (XOS) ; les chitosan-oligosaccharides (COS) ; un α-galactoside choisi parmi le raffinose, le stachyose ; les pectines ; les gommes ; les gommes partiellement hydrolysées ; l'inuline ; le psyllium ; les arabinogalactanes ; les fibres d'acacia, de caroube, d'avoine, de bambou et d'agrumes.

6. Formulation contenant une composition sous forme solide pour son utilisation selon l'une ou plusieurs des revendications 1 à 5, et un milieu liquide hydrophile.

7. Formulation contenant la composition sous forme solide pour son utilisation selon la revendication 6, dans laquelle l'agent viscosifiant est présent en un pourcentage supérieur ou égal à 0,05 % (p/v), sur la base du volume final de la formulation ; de préférence compris entre 0,2 et 20 % (p/v).
